(19) Europäisches Patentamt
European Patent Office
Office européen des brevets

(11) EP 4 659 730 A1

(12) # EUROPEAN PATENT APPLICATION

published in accordance with Art. 153(4) EPC

(43) Date of publication:
**10.12.2025 Bulletin 2025/50**

(21) Application number: **24750414.5**

(22) Date of filing: **02.02.2024**

(51) International Patent Classification (IPC):
***A61K 6/54*** *(2020.01)* ***A61K 8/19*** *(2006.01)*
***A61K 8/24*** *(2006.01)* ***A61K 8/37*** *(2006.01)*
***A61Q 11/00*** *(2006.01)*

(52) Cooperative Patent Classification (CPC):
**A61K 6/54; A61K 8/19; A61K 8/24; A61K 8/37; A61Q 11/00**

(86) International application number:
**PCT/JP2024/003438**

(87) International publication number:
**WO 2024/162465 (08.08.2024 Gazette 2024/32)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **02.02.2023 JP 2023014867**

(71) Applicant: **Kuraray Noritake Dental Inc.**
**Kurashiki-shi, Okayama 710-0801 (JP)**

(72) Inventor: **KAWASHIMA, Mitsunobu**
**Tainai-shi, Niigata 959-2653 (JP)**

(74) Representative: **D Young & Co LLP**
**3 Noble Street**
**London EC2V 7BQ (GB)**

(54) **DENTAL COMPOSITION**

(57) The present invention relates to a dental composition containing a compound (a) having an acidic group represented by the following formula (I): $R^1$-$X_k$ (I), in which in the formula (I), $R^1$ represents an organic group having 40 or less carbon atoms that has or does not have at least one (meth)acryloyloxy group, X represents an acidic group, and k represents an integer of 1 or 2, in which in the case where k is 2, two acidic groups X may be the same as or different from each other, a calcium ion (b1), water (c), and a hydrophilic solvent (d), substantially not containing a crosslinkable polymerizable monomer that is an organic compound other than the compound (a) having an acidic group, and is an organic compound other than the hydrophilic solvent (d).

EP 4 659 730 A1

## Description

### Technical Field

The present invention relates to a dental composition.

### Background Art

In recent years, the so-called "8020 Campaign", which encourages people to keep 20 or more of their own teeth even at age 80, is leading to an increase in the number of remaining teeth of the elderly. However, the remaining teeth of the elderly are at risk of gingival recession due to periodontal disease or the like, resulting in exposure of the tooth root. The exposed root surface is susceptible to caries. Accordingly, the increase in the number of remaining teeth of the elderly results in the problem of root surface caries, which is specific to the elderly, due to the exposure of the tooth root. Therefore, combined with the increase in the elderly population, the treatment of the root surface caries has become an urgent issue.

Under the circumstances, dental compositions have been investigated and developed from the standpoint of the suppression of the dental caries progression and the prevention of the dental caries occurrence.

For example, PTL 1 describes a dental material containing a monomeric calcium salt ($S_A$) including a monomer (A) having in the molecule thereof a polymerizable group and at least one acidic group selected from the group consisting of a carboxylic acid group and a phosphoric acid group, in which the acidic group is substantially completely neutralized with calcium, and/or a polymeric calcium salt ($S_P$), which is a polymer (P) of a polymerizable monomer containing a monomer (A) having in the molecule thereof a polymerizable group and at least one acidic group selected from the group consisting of a carboxylic acid group and a phosphoric acid group, in which the acidic group is substantially completely neutralized with calcium.

PTL 2 describes a two-component dental adhesive including a component A containing a first polymerizable monomer having a free acidic group and/or an acidic group forming an acid anhydride, and a component B containing a second polymerizable monomer having an acidic group forming a calcium salt.

### Citation List

### Patent Literatures

PTL 1: JP 2006-347943 A
PTL 2: JP 2019-99481 A

### Summary of Invention

### Technical Problem

The root surface caries is a dental disease that has no suitable treatment method established therefor, and a novel material that is suitable for the disease has been demanded, and for the prevention of the root surface caries, it is important to suppress the demineralization by enhancing the acid resistance of the tooth substance, particularly the dentin, and to suppress the decomposition and dissolution of collagen, an organic component thereof, by imparting base resistance thereto.

In view of the problem, an object of the present invention is to provide a dental composition that is excellent in sealability of the tooth substance, particularly the dentin, and is capable of imparting favorable acid resistance and base resistance to the tooth substance surface.

### Solution to Problem

The present inventors have made earnest investigations for solving the problem, it has been found that the problem can be solved by coating, on the tooth surface, a particular composition containing a compound having an acidic group, a calcium compound, water, and a hydrophilic solvent, and thus the present invention has been completed.

Specifically, the present invention encompasses the following inventions.

[1] A dental composition containing

a compound (a) having an acidic group represented by the following formula (I):

$$R^1\text{-}X_k \qquad (I)$$

in which in the formula (I), $R^1$ represents an organic group having 40 or less carbon atoms that has or does not have at least one (meth)acryloyloxy group, X represents an acidic group, and k represents an integer of 1 or 2, in which in the case where k is 2, two acidic groups X may be the same as or different from each other,
a calcium ion (b1), water (c), and a hydrophilic solvent (d),
substantially not containing a crosslinkable polymerizable monomer that is an organic compound other than the compound (a) having an acidic group, and is an organic compound other than the hydrophilic solvent (d).

[2] The dental composition according to the item [1], in which the compound (a) having an acidic group is represented by the following formula (II):

$$R^1\text{-}((A^{m-})_a\cdot(H^+)_b)_k \qquad (II)$$

in which in the formula (II), $R^1$ and k have the same meanings as defined in the formula (I), $A^{m-}$ represents an m-valent acid anion group derived from the acidic group X, m represents an integer of 1, 2, or 3, and a and b represent integers of 1, 2, or 3 that have a relationship of am = b.
[3] The dental composition according to the item [1] or [2], in which the compound (a) having an acidic group is represented by the following formula (III):

$$R^2\text{-}R^3\text{-O-P(=O)(-OH)}_2 \qquad (III)$$

in which in the formula (III), $R^2$ represents a (meth)acryloyloxy group or hydrogen, and $R^3$ represents an alkylene group having 6 to 20 carbon atoms.
[4] The dental composition according to any one of the items [1] to [3], in which the calcium ion (b1) is derived from at least one calcium compound (b) selected from the group consisting of CaO and a compound represented by the following formula (IV):

$$(Ca^{2+})_c\cdot(Y^{n-})_d \qquad (IV)$$

in which in the formula (IV), $Y^{n-}$ represents an anion as a counter ion of the calcium ion, n represents an integer of 1, 2, or 3, and c and d each represent an integer of 1, 2, or 3, in which c, d, and n have a relationship satisfying an expression 2c = nd.
[5] The dental composition according to any one of the items [1] to [4], in which the calcium ion (b1) is derived from at least one calcium compound (b) selected from the group consisting of CaO, $CaCl_2$, $Ca(OH)_2$, and $CaF_2$.
[6] The dental composition according to any one of the items [1] to [5], in which the dental composition has a content of the component (c) of 1 to 90% by mass and a content of the component (d) of 1 to 90% by mass based on 100% by mass in total of the compound (a) having an acidic group, the water (c), and the hydrophilic solvent (d).
[7] The dental composition according to any one of the items [1] to [6], in which the dental composition has a content of the component (a) of 10 to 70% by mass, a content of the component (c) of 1 to 90% by mass, and a content of the component (d) of 1 to 90% by mass, based on 100% by mass in total of the compound (a) having an acidic group, the water (c), and the hydrophilic solvent (d).
[8] The dental composition according to any one of the items [1] to [7], in which the dental composition has a ratio of a base gram equivalent derived from the calcium compound (b) with respect to an acid gram equivalent derived from the compound (a) having an acidic group (base gram equivalent/acid gram equivalent) of less than 0.50.
[9] The dental composition according to any one of the items [1] to [8], in which the dental composition substantially does not contain a zinc ion.
[10] The dental composition according to any one of the items [1] to [9], in which the dental composition is a dentin strengthening agent.

Advantageous Effects of Invention

The present invention can provide a dental composition that is excellent in sealability of the tooth substance, particularly the dentin, and is capable of imparting favorable acid resistance and base resistance to the tooth substance surface.

Description of Embodiments

The present invention will be described in detail with reference to embodiments below.

In the description herein, the upper limit values and the lower limit values of the numerical ranges (e.g., the contents of the components, and the values and the properties calculated from the components) can be optionally combined.

Specifically, in the description herein, the upper limit values and the lower limit values described in a stepwise manner for the numerical ranges can each be independently combined. For example, with reference to the description "preferably 10 to 90, and more preferably 30 to 60" for the same item, a range of "10 to 60" can be derived from the "preferred lower limit value (10)" and the "more preferred upper limit value (60)".

For the numerical ranges, for example, based on the description "preferably 10 to 90, and more preferably 30 to 60", only the lower limit value can be defined as "10 or more" or "30 or more" with no particular definition of the upper limit value. Similarly, only the upper limit value can be defined as "90 or less" or "60 or less" with no particular definition of the lower limit value.

A numerical range simply defined as "10 to 90" means a range of 10 or more and 90 or less unless otherwise indicated.

As similar to as described above, with reference to the description "preferably 10 or more, and more preferably 30 or more" and the description "preferably 90 or less, and more preferably 60 or less" for the same item, a range of "10 or more and 60 or less" can be derived from the "preferred lower limit value (10)" and the "more preferred upper limit value (60)". As similar to as described above, only the lower limit value can be defined as "10 or more" or "30 or more", and similarly, only the upper limit value can be defined as "90 or less" or "60 or less". The same is applied to the case where the upper end of the numerical range is "less than" and the case where the lower end thereof is "more than".

In the description herein, the expression "(meth)acrylic" is used as meaning encompassing both "methacrylic" and "acrylic". The same is applied to the similar expressions, such as "(meth)acrylate", "(meth)acrylamide", and "(meth) acryloyloxy".

[Dental Composition]

The dental composition according to one embodiment of the present invention (which may be hereinafter referred to as a "dental composition") contains a compound (a) having an acidic group (which may be hereinafter referred to as a "component (a)") represented by the following formula (I):

$$R^1\text{-}X_k \qquad (I)$$

in which in the formula (I), $R^1$ represents an organic group having 40 or less carbon atoms that has or does not have at least one (meth)acryloyloxy group, X represents an acidic group, and k represents an integer of 1 or 2, in which in the case where k is 2, two acidic groups X may be the same as or different from each other,

a calcium ion (b1) (which may be hereinafter referred to as a "component (b1)"), water (c) (which may be hereinafter referred to as a "component (c)"), and a hydrophilic solvent (d) (which may be hereinafter referred to as a "component (d)"), and

substantially does not contain a crosslinkable polymerizable monomer that is an organic compound other than the compound (a) having an acidic group, and is an organic compound other than the hydrophilic solvent (d).

The present inventors estimate the mechanism that the dental composition provides the effects of the present invention as follows while not limited thereto.

By applying the dental composition to a tooth substance surface, a deposit is formed on the coated tooth substance surface. It is considered that the deposit is a substance formed in such a manner that the acidic mixture formed of the compound (a) having an acidic group, the water (c), and the hydrophilic solvent (d) demineralizes the smear layer on the surface layer of the tooth substance and the surface layer portion of the tooth substance and penetrates into the tooth substance, and in this process, the compound (a) having an acidic group, the calcium ion (b1), and the inorganic component of the demineralized tooth substance are precipitated through compounding, and deposited on the tooth substance surface. It is estimated that the dental composition forms the deposit, and thereby exerts excellent sealability of the tooth substance and imparts favorable acid resistance and base resistance to the tooth substance surface.

In the description herein, the functions of providing the sealability of the tooth substance and imparting the favorable acid resistance and base resistance to the tooth substance surface, which are achieved by the dental composition, may be collectively referred to as "tooth substance strengthening".

The components contained in the dental composition will be described below.

<Compound (a) having Acidic Group>

The compound (a) having an acidic group is represented by the following formula (I):

$$R^1\text{-}X_k \qquad (I)$$

in which in the formula (I), $R^1$ represents an organic group having 40 or less carbon atoms that has or does not have at least one (meth)acryloyloxy group, X represents an acidic group, and k represents an integer of 1 or 2, in which in the case where k is 2, two acidic groups X may be the same as or different from each other.

$R^1$ represents an organic group having 40 or less carbon atoms having at least one (meth)acryloyloxy group or an organic group having 40 or less carbon atoms having no (meth)acryloyloxy group, in which the number of carbon atoms of the organic group is not particularly limited, as long as exerting the effects of the present invention, and is 1 to 40, preferably 2 to 35, more preferably 4 to 30, further preferably 5 to 25, and still further preferably 6 to 20.

Examples of the acidic group represented by X include a phosphoric acid group, a pyrophosphoric acid group, a thiophosphoric acid group, a phosphonic acid group, a sulfonic acid group, and a carboxylic acid group. Among these, a phosphoric acid group and a pyrophosphoric acid group are preferred, and a phosphoric acid group is more preferred.

The compound (a) having an acidic group represented by the formula (I) is a compound having an acidic group that allows the acidic composition obtained by mixing the water (c) and the hydrophilic solvent (d) to exert the acid etching effect and the primer treatment effect to the tooth substance, and is a component that imparts the demineralization function and the penetration function.

The compound (a) having an acidic group represented by the formula (I) is preferably represented by the following formula (II):

$$R^1\text{-}((A^{m-})_a \cdot (H^+)_b)_k \qquad (II)$$

in which in the formula (II), $R^1$ and k have the same meanings as defined in the formula (I), $A^{m-}$ represents an m-valent acid anion group derived from the acidic group X, m represents an integer of 1, 2, or 3, and a and b represent integers of 1, 2, or 3 that have a relationship of am = b.

Examples of the compound (a) having an acidic group include a compound having at least one acidic group, such as a phosphoric acid group, a pyrophosphoric acid group, a thiophosphoric acid group, a phosphonic acid group, a sulfonic acid group, and a carboxylic acid group. Specific examples of the compound (a) having an acidic group are shown below.

Examples of the phosphoric acid group-containing compound include octyl dihydrogen phosphate, decyl dihydrogen phosphate, dodecyl dihydrogen phosphate, tetradecyl dihydrogen phosphate, hexadecyl dihydrogen phosphate, octadecyl dihydrogen phosphate, 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, bis[2-(meth)acryloyloxyethyl] hydrogen phosphate, bis[4-(meth)acryloyloxybutyl] hydrogen phosphate, bis[6-(meth)acryloyloxyhexyl] hydrogen phosphate, bis[8-(meth)acryloyloxyoctyl] hydrogen phosphate, bis[9-(meth)acryloyloxynonyl] hydrogen phosphate, bis[10-(meth)acryloyloxydecyl] hydrogen phosphate, 1,3-di(meth)acryloyloxypropyl dihydrogen phosphate, 2-(meth)acryloyloxyethylphenyl hydrogen phosphate, 2-(meth)acryloyloxyethyl-2-bromoethyl hydrogen phosphate, and bis[2-(meth)acryloyloxy-(1-hydroxymethyl)ethyl] hydrogen phosphate.

Examples of the pyrophosphoric acid group-containing compound include bis[2-(meth)acryloyloxyethyl] pyrophosphate, bis[4-(meth)acryloyloxybutyl] pyrophosphate, bis[6-(meth)acryloyloxyhexyl] pyrophosphate, bis[8-(meth)acryloyloxyoctyl] pyrophosphate, and bis[10-(meth)acryloyloxydecyl] pyrophosphate.

Examples of the thiophosphoric acid group-containing compound include 2-(meth)acryloyloxyethyl dihydrogen thiophosphate, 3-(meth)acryloyloxypropyl dihydrogen thiophosphate, 4-(meth)acryloyloxybutyl dihydrogen thiophosphate, 5-(meth)acryloyloxypentyl dihydrogen thiophosphate, 6-(meth)acryloyloxyhexyl dihydrogen thiophosphate, 7-(meth)acryloyloxyheptyl dihydrogen thiophosphate, 8-(meth)acryloyloxyoctyl dihydrogen thiophosphate, 9-(meth)acryloyloxynonyl dihydrogen thiophosphate, 10-(meth)acryloyloxydecyl dihydrogen thiophosphate, 11-(meth)acryloyloxyundecyl dihydrogen thiophosphate, 12-(meth)acryloyloxydodecyl dihydrogen thiophosphate, 16-(meth)acryloyloxyhexadecyl dihydrogen thiophosphate, and 20-(meth)acryloyloxyicosyl dihydrogen thiophosphate.

Examples of the phosphonic acid group-containing compound include 2-(meth)acryloyloxyethyl phenyl phosphonate, 5-(meth)acryloyloxypentyl-3-phosphonopropionate, 6-(meth)acryloyloxyhexyl-3-phosphonopropionate, 10-(meth)acryloyloxydecyl-3-phosphonopropionate, 6-(meth)acryloyloxyhexyl phosphonoacetate, and 10-(meth)acryloyloxydecyl phosphonoacetate.

Examples of the sulfonic acid group-containing compound include 2-(meth)acrylamido-2-methylpropanesulfonic acid, styrenesulfonic acid, and 2-sulfoethyl (meth)acrylate.

Examples of the carboxylic acid group-containing compound include a compound having one carboxy group in a molecule and a compound having multiple carboxy groups in a molecule.

Examples of the compound having one carboxy group in a molecule include (meth)acrylic acid, N-(meth)acryloylglycine, N-(meth)acryloylaspartic acid, O-(meth)acryloyltyrosine, N-(meth)acryloyltyrosine, N-(meth)acryloylphenylalanine, N-(meth)acryloyl-p-aminobenzoic acid, N-(meth)acryloyl-o-aminobenzoic acid, p-vinylbenzoic acid, 2-(meth)acryloyloxybenzoic acid, 3-(meth)acryloyloxybenzoic acid, 4-(meth)acryloyloxybenzoic acid, N-(meth)acryloyl-5-aminosalicylic acid, N-(meth)acryloyl-4-aminosalicylic acid, 2-(meth)acryloyloxyethyl hydrogen succinate, 2-(meth)acryloyloxyethyl hydrogen phthalate, and 2-(meth)acryloyloxyethyl hydrogen maleate.

Examples of the compound having multiple carboxy groups in a molecule include 6-(meth)acryloyloxyhexane-1,1-dicarboxylic acid, 9-(meth)acryloyloxynonane-1,1-dicarboxylic acid, 10-(meth)acryloyloxydecane-1,1-dicarboxylic acid, 11-(meth)acryloyloxyundecane-1,1-dicarboxylic acid, 12-(meth)acryloyloxydodecane-1,1-dicarboxylic acid, 13-(meth)acryloyloxytridecane-1,1-dicarboxylic acid, 4-(meth)acryloyloxyethyl trimellitate, 4-(meth)acryloyloxybutyl trimellitate, 4-(meth)acryloyloxyhexyl trimellitate, 4-(meth)acryloyloxydecyl trimellitate, and 2-(meth)acryloyloxyethyl-3'-(meth)acryloyloxy-2'-(3,4-dicarboxybenzoyloxy)propyl succinate.

The compound (a) having an acidic group may be a compound represented by the following formula (III):

$$R^2\text{-}R^3\text{-O-P(=O)(-OH)}_2 \qquad \text{(III)}$$

in which in the formula (III), $R^2$ represents a (meth)acryloyloxy group or hydrogen, and $R^3$ represents an alkylene group having 6 to 20 carbon atoms.

In the compound (a) having an acidic group, a phosphoric acid group-containing compound, a pyrophosphoric acid group-containing compound, and a carboxylic acid group-containing compound are preferred since a better deposit formation effect is exerted thereby on the tooth substance, in which a phosphoric acid group-containing compound and a carboxylic acid group-containing compound are more preferred, and dodecyl dihydrogen phosphate, hexadecyl dihydrogen phosphate, 6-(meth)acryloyloxyhexyl dihydrogen phosphate, 10-(meth)acryloyloxydecyl dihydrogen phosphate, 12-(meth)acryloyloxydodecyl dihydrogen phosphate, and 4-(meth)acryloyloxyethyl trimellitate are further preferred. Among these, from the standpoint of further facilitating the formation of the deposit, a divalent phosphoric acid group-containing compound having an alkyl group having 6 to 20 carbon atoms as a main chain in a molecule, and a divalent phosphoric acid group-containing compound having an alkylene group having 6 to 20 carbon atoms as a main chain in a molecule, such as the compound represented by the formula (III), are still further preferred, and a divalent phosphoric acid group-containing compound having an alkyl group having 6 to 12 carbon atoms as a main chain in a molecule, such as dodecyl dihydrogen phosphate, hexadecyl dihydrogen phosphate, 6-(meth)acryloyloxyhexyl dihydrogen phosphate, 10-(meth)acryloyloxydecyl dihydrogen phosphate, and 12-(meth)acryloyloxydodecyl dihydrogen phosphate, and a divalent phosphoric acid group-containing compound having an alkylene group having 6 to 20 carbon atoms as a main chain in a molecule are still more further preferred.

One kind of the compound (a) having an acidic group may be used alone, or two or more kinds thereof may be used in combination. The content of the compound (a) having an acidic group is preferably 10% by mass or more, more preferably 15% by mass or more, and further preferably 20% by mass or more, based on 100% by mass in total of the components (a), (c), and (d), from the standpoint of facilitating the securement of the effects of the present invention. The content thereof is preferably 70% by mass or less, more preferably 60% by mass or less, and further preferably 50% by mass or less. As described above, the lower limit values and the upper limit values described in a stepwise manner each can be independently combined. For example, in one embodiment of the present invention, the content of the component (a) is preferably 10 to 70% by mass, more preferably 15 to 60% by mass, and further preferably 20 to 50% by mass, based on 100% by mass in total of the components (a), (c), and (d).

<Calcium Ion (b1) and Calcium Compound (b)>

The calcium ion (b1) is derived from the calcium compound (b). The calcium compound (b) is dissolved with the compound (a) having an acidic group, so as to allow the dental composition to contain the calcium ion (b1).

The calcium ion (b1) is preferably derived from at least one calcium compound (b) selected from the group consisting of CaO and a compound represented by the following formula (IV):

$$(Ca^{2+})_c \cdot (Y^{n-})_d \qquad \text{(IV)}$$

wherein in the formula (IV), $Y^{n-}$ represents an anion as a counter ion of the calcium ion, n represents an integer of 1, 2, or 3, and c and d each represent an integer of 1, 2, or 3, in which c, d, and n have a relationship satisfying an expression 2c = nd.

Specific examples of the calcium compound (b) include at least one calcium compound (b) selected from the group consisting of CaO, $CaCl_2$, $Ca(OH)_2$, and $CaF_2$, and more preferred examples thereof include at least one selected from the group consisting of CaO, $CaCl_2$, and $Ca(OH)_2$.

One kind of the calcium compound (b) used for preparing the dental composition may be used alone, or two or more kinds thereof may be used in combination.

The content of the calcium ion (b1) blended in the dental composition is preferably 0.1 part by mass or more, more preferably 0.3 part by mass or more, further preferably 0.6 part by mass or more, still further preferably 0.7 part by mas or more, and still more further preferably 0.8 part by mass or more, from the standpoint of facilitating the favorable formation of the deposit for strengthening the tooth substance, and is preferably 10 parts by mass or less, more preferably 7.0 parts by mass or less, further preferably 5.0 parts by mass or less, still further preferably 4.0 parts by mass or less, and still more further preferably 3.0 parts by mass or less, from the standpoint of suppressing the neutralization of the acidic group of the component (a) from proceeding excessively, and securing the favorable demineralization function and penetration function, per 100 parts by mass in total of the components (a), (c), and (d). As described above, the lower limit values and the upper limit values described in a stepwise manner each can be independently combined. For example, in one embodiment of the present invention, the content of the component (b1) is preferably 0.1 to 10 parts by mass, more preferably 0.3 to 7.0 parts by mass, further preferably 0.6 to 5.0 parts by mass, still further preferably 0.7 to 4.0 parts by mass, and still more further preferably 0.8 to 3.0 parts by mass, per 100 parts by mass in total of the components (a), (c), and (d).

The blending amount of the calcium compound (b) used in preparing the dental composition is determined by the intended content of the calcium ion (b1) in the dental composition and the molar mass of the selected calcium compound (b), i.e., can be obtained by the following expression.

Blending amount (g) of calcium compound (b) = (molar mass of calcium compound (b)) (g/mol) / (molar mass of calcium) (g/mol) × (content of calcium ion (b1)) (g)

For example, in the case where $Ca(OH)_2$ is used as the calcium compound (b), the molar mass of $Ca(OH)_2$ is 74.1 g/mol, and the molar mass of calcium is 40.1 g/mol. The preferred blending amount of $Ca(OH)_2$ is, corresponding to the intended content of the calcium ion (b1), for example, 0.2 part by mass or more, more preferably 0.6 part by mass or more, further preferably 1.1 parts by mass or more, still further preferably 1.3 parts by mass or more, and still more further preferably 1.5 parts by mass or more, and from the standpoint of suppressing the neutralization of the acidic group of the component (a) from proceeding excessively, and securing the favorable demineralization function and penetration function, is preferably 19 parts by mass or less, more preferably 13 parts by mass or less, further preferably 9.2 parts by mass or less, still further preferably 7.4 parts by mass or less, and still more further preferably 5.5 parts by mass or less, per 100 parts by mass in total of the components (a), (c), and (d). As described above, the lower limit values and the upper limit values described in a stepwise manner each can be independently combined. For example, in one embodiment of the present invention, in the case where $Ca(OH)_2$ is used as the calcium compound (b), the preferred blending amount of $Ca(OH)_2$ is, corresponding to the intended content of the calcium ion (b1), for example, 0.2 to 19 parts by mass, more preferably 0.6 to 13 parts by mass, further preferably 1.1 to 9.2 parts by mass, still further preferably 1.3 to 7.4 parts by mass, and still more further preferably 1.5 to 5.5 parts by mass, per 100 parts by mass in total of the components (a), (c), and (d).

For example, in the case where $CaCl_2$ is used as the calcium compound (b), the molar mass of $CaCl_2$ is 111 g/mol. The preferred blending amount of $CaCl_2$ is, corresponding to the intended content of the calcium ion (b1), for example, 0.3 part by mass or more, more preferably 0.8 part by mass or more, further preferably 1.7 parts by mass or more, still further preferably 1.9 parts by mass or more, and still more further preferably 2.2 parts by mass or more, and from the standpoint of suppressing the neutralization of the acidic group of the component (a) from proceeding excessively, and securing the favorable demineralization function and penetration function, is preferably 28 parts by mass or less, more preferably 19.4 parts by mass or less, further preferably 13.8 parts by mass or less, still further preferably 11.1 parts by mass or less, and still more further preferably 8.3 parts by mass or less, per 100 parts by mass in total of the components (a), (c), and (d). As described above, the lower limit values and the upper limit values described in a stepwise manner each can be independently combined. For example, in one embodiment of the present invention, in the case where $CaCl_2$ is used as the calcium compound (b), the preferred blending amount of $CaCl_2$ is, corresponding to the intended content of the calcium ion (b1), for example, 0.3 to 28 parts by mass, more preferably 0.8 to 19.4 parts by mass, further preferably 1.7 to 13.8 parts by mass, still further preferably 1.9 to 11.1 parts by mass, and still more further preferably 2.2 to 8.3 parts by mass, per 100 parts by mass in total of the components (a), (c), and (d).

For example, in the case where CaO is used as the calcium compound (b), the molar mass of CaO is 56.1 g/mol. The preferred blending amount of CaO is, corresponding to the intended amount of the calcium ion (b1), for example, 0.1 part by mass or more, more preferably 0.4 part by mass or more, further preferably 0.8 parts by mass or more, still further

preferably 1.0 parts by mass or more, and still more further preferably 1.1 parts by mass or more, and from the standpoint of suppressing the neutralization of the acidic group of the component (a) from proceeding excessively, and securing the favorable demineralization function and penetration function, is preferably 14 parts by mass or less, more preferably 9.8 parts by mass or less, further preferably 7.0 parts by mass or less, still further preferably 5.6 parts by mass or less, and still more further preferably 4.2 parts by mass or less, per 100 parts by mass in total of the components (a), (c), and (d). As described above, the lower limit values and the upper limit values described in a stepwise manner each can be independently combined. For example, in one embodiment of the present invention, in the case where CaO is used as the calcium compound (b), the preferred blending amount of CaO is, corresponding to the intended amount of the calcium ion (b1), for example, 0.1 to 14 parts by mass, more preferably 0.4 to 9.8 parts by mass, further preferably 0.8 to 7.0 parts by mass, still further preferably 1.0 to 5.6 parts by mass, and still more further preferably 1.1 to 4.2 parts by mass, per 100 parts by mass in total of the components (a), (c), and (d).

In the description herein, the content of the calcium ion (b1) in the dental composition can be calculated from the blending amount of the calcium compound (b) generating the calcium ion (b1), and can also be obtained through the measurement with an ICP emission spectrometer.

In the dental composition according to one embodiment of the present invention, the ratio of the base gram equivalent derived from the calcium compound (b) with respect to the acid gram equivalent derived from the compound (a) having an acidic group (base gram equivalent/acid gram equivalent) is preferably 0.01 or more, more preferably 0.05 or more, further preferably 0.08 or more, and still further preferably 0.10 or more, from the standpoint of facilitating the effects of the present invention. The ratio (base gram equivalent/acid gram equivalent) is preferably less than 0.50, more preferably 0.40 or less, further preferably 0.35 or less, and still further preferably 0.30 or less, from the standpoint of the demineralization function of the dental composition to the tooth substance, while there is no limitation, as long as exerting the effects of the present invention. As described above, the lower limit values and the upper limit values described in a stepwise manner each can be independently combined. For example, in one embodiment of the present invention, the ratio (base gram equivalent/acid gram equivalent) is preferably 0.01 or more and less than 0.50, more preferably 0.05 to 0.40, further preferably 0.08 to 0.35, and still further preferably 0.10 to 0.30.

The gram equivalent herein means a value obtained by multiplying the molar number by the valence of the acid (or the base), or in other words, means the molar number of $H^+$ (or $OH^-$) that the acid (or the base) can generate in neutralization.

In the description herein, the base gram equivalent derived from the calcium compound (b) in the dental composition can be calculated based on the blending amount of the calcium compound (b) generating the calcium ion (b1), and can also be calculated from the content of the calcium ion (b1) obtained through the measurement with an ICP emission spectrometer.

The acid gram equivalent derived from the compound (a) having an acidic group in the dental composition can be calculated based on the blending amount of the compound (a) having an acidic group.

<Water (c)>

The water (c) is preferably distilled water or ion exchanged water. The content of the water (c) in the dental composition is preferably 1 to 90% by mass, more preferably 5 to 80% by mass, further preferably 10 to 70% by mass, and still further preferably 15 to 45% by mass, based on 100% by mass in total of the components (a), (c), and (d).

<Hydrophilic Solvent (d)>

The hydrophilic solvent (d) means an organic compound that has a solubility of 11 g or more in 100 g of water at 25°C, and is preferably one having the solubility that is 43 g or more.

The hydrophilic solvent (d) may be a single compound or a mixture of multiple compounds.

Examples of the hydrophilic solvent (d) include ethanol, 2-propanol, 2-methyl-2-propanol, acetone, tetrahydrofuran, 2-hydroxyethyl (meth)acrylate, 3-hydroxypropyl (meth)acrylate, 2-hydroxypropyl (meth)acrylate, 1,3-dihydroxypropyl (meth)acrylate, 2,3-dihydroxypropyl (meth)acrylate, 2-((meth)acryloyloxy)ethyltrimethylammonium chloride, polyethylene glycol di(meth)acrylate (in which the number of oxyethylene groups is 9 or more), N,N-dimethylaminoethyl (meth)acrylate, N-methylol(meth)acrylamide, N-hydroxyethyl(meth)acrylamide, N-methoxymethyl(meth)acrylamide, N-ethoxymethyl(meth)acrylamide, diacetone(meth)acrylamide, 4-(meth)acryloylmorpholine, N-[tris(hydroxymethyl)methyl](meth)acrylamide, N-[3-(dimethylamino)propyl](meth)acrylamide, and a (meth)acrylamide represented by the following formula (V):

[Chem. 1]

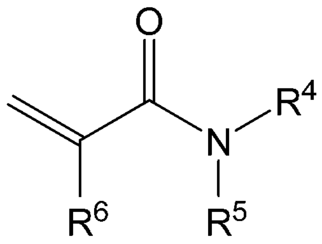

(V)

wherein in the formula (V), $R^4$ and $R^5$ each independently represent an alkyl group having 1 to 3 carbon atoms (such as a methyl group, an ethyl group, a n-propyl group, and an isopropyl group), which may have a substituent (such as a hydroxy group), and $R^6$ represents a hydrogen atom or a methyl group.

In the hydrophilic solvent (d), ethanol, 2-propanol, 2-methyl-2-propanol, acetone, tetrahydrofuran, 2-hydroxyethyl (meth)acrylate, 2,3-dihydroxypropyl (meth)acrylate, N,N-dimethylaminoethyl (meth)acrylate, diacetone(meth) acrylamide, N-[tris(hydroxymethyl)methyl](meth)acrylamide, and a (meth)acrylamide represented by the formula (V) are preferred, and ethanol, 2-propanol, 2-hydroxyethyl (meth)acrylate, N,N-dimethylaminoethyl (meth)acrylate, and a (meth) acrylamide represented by the formula (V) are more preferred.

Specific examples of the (meth)acrylamide represented by the formula (V) include N,N-dimethyl(meth)acrylamide, N,N-diethyl(meth)acrylamide, and N,N-bis(2-hydroxyethyl)(meth)acrylamide. In one embodiment of the present invention, in the specific examples of the (meth)acrylamide represented by the formula (V), N,N-dimethylacrylamide and N,N-diethylacrylamide are preferred, and N,N-diethylacrylamide is more preferred, from the standpoint of achieving better storage stability.

One kind of the hydrophilic solvent (d) may be blended alone, or two or more kinds thereof may be used in combination.

The hydrophilic solvent (d) functions as a cosolvent of the compound (a) having an acidic group and the water (c). The content of the hydrophilic solvent (d) in the dental composition is preferably 1 to 90% by mass, more preferably 5 to 80% by mass, further preferably 10 to 70% by mass, and still further preferably 20 to 50% by mass, based on 100% by mass in total of the compound (a) having an acidic group, the water (c), and the hydrophilic solvent (d).

In the dental composition, the total content (% by mass) of the compound (a) having an acidic group, the water (c), and the hydrophilic solvent (d) is preferably 90 to 99% by mass, more preferably 93 to 99% by mass, and further preferably 95 to 99% by mass, based on 100% by mass of the dental composition, from the standpoint of further facilitating the effects of the present invention.

In the dental composition, the total content (% by mass) of the compound (a) having an acidic group, the calcium compound (b) generating the calcium ion (b1), the water (c), and the hydrophilic solvent (d) is preferably 90 to 100% by mass, more preferably 94 to 100% by mass, further preferably 98 to 100% by mass, and still further preferably 99 to 100% by mass, based on 100% by mass of the dental composition, from the standpoint of further facilitating the effects of the present invention.

<Additional Organic Compound>

The dental composition may contain an organic compound that is an organic compound other than the compound (a) having an acidic group and other than the hydrophilic solvent (d) (which may be hereinafter referred to as an "additional organic compound"). However, the dental composition substantially does not contain a crosslinkable polymerizable monomer that is the additional organic compound (which may be hereinafter referred to as a "crosslinkable polymerizable monomer"). The expression "substantially not containing" herein specifically means that the amount of the crosslinkable polymerizable monomer is 5% by mass or less, preferably 3% by mass or less, more preferably 1% by mass or less, still further preferably 0.1% by mass or less, and still more further preferably 0.05% by mass or less, based on 100% by mass of the dental composition. In other words, in one embodiment of the present invention, the content of the crosslinkable polymerizable monomer is 0 to 5% by mass, preferably 0 to 3% by mass, more preferably 0 to 1% by mass, further preferably 0 to 0.1% by mass, and still further preferably 0 to 0.05% by mass, based on 100% by mass of the dental composition.

Examples of the crosslinkable polymerizable monomer include an aromatic bifunctional monomer, an aliphatic bifunctional monomer, and a trifunctional or higher functional monomer, which are hydrophobic crosslinkable polymerizable monomers having no acidic group.

The dental composition according to an embodiment of the present invention substantially does not contain a crosslinkable polymerizable monomer, which thereby prevents the crosslinkable polymerizable monomer from inhibiting the function of compounding the compound (a) having an acidic group, the calcium ion (b1), and the inorganic component of the demineralized tooth substance, accelerating the formation of the deposit thereby.

Examples of the aromatic bifunctional monomer include 2,2-bis((meth)acryloyloxyphenyl)propane, 2,2-bis[4-(3-(meth)acryloyloxy-2-hydroxypropoxy)phenyl]propane, 2,2-bis(4-(meth)acryloyloxyethoxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, and 2,2-bis(4-(meth)acryloyloxyisopropoxyphenyl)propane.

Examples of the aliphatic bifunctional monomer include glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, 1,3-butanediol di(meth)acrylate, 1,5-pentanediol di(meth)acrylate, 1,6-hexanediol di(meth)acrylate, 1,10-decanediol di(meth)acrylate, 1,2-bis(3-methacryloyloxy-2-hydroxypropoxy)ethane, 2,2,4-trimethylhexamethylene bis(2-carbamoyloxyethyl) di(meth)acrylate, N-methacryloyloxyethylacrylamide, N-methacryloyloxypropylacrylamide, N-methacryloyloxybutylacrylamide, N-(1-ethyl-(2-methacryloyloxy)ethyl)acrylamide, and N-(2-(2-methacryloyloxyethoxy)ethyl)acrylamide.

Examples of the trifunctional or higher functional monomer include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol penta(meth)acrylate, N,N-(2,2,4-trimethylhexamethylene)bis[2-(aminocarboxy)propane-1,3-diol] tetra(meth)acrylate, and 1,7-diacryloyloxy-2,2,6,6-tetra(meth)acryloyloxymethyl-4-oxyheptane.

The dental composition may contain a substance that is the additional organic compound and is other than the crosslinkable polymerizable monomer, and preferably substantially does not contain the substance. The expression "substantially does not contain" herein specifically means that the amount of the substance that is the additional organic compound and is other than the crosslinkable polymerizable monomer is 5% by mass or less, preferably 3% by mass or less, more preferably 1% by mass or less, still further preferably 0.1% by mass or less, and still more further preferably 0.05% by mass or less, based on 100% by mass of the dental composition. In other words, in the description herein, the expression "substantially does not contain the additional organic compound other than the crosslinkable polymerizable monomer" means that the content of the additional organic compound other than the crosslinkable polymerizable monomer is 0 to 5% by mass, preferably 0 to 3% by mass, more preferably 0 to 1% by mass, further preferably 0 to 0.1% by mass, and still further preferably 0 to 0.05% by mass, based on 100% by mass of the dental composition.

Examples of the substance that is the additional organic compound and is other than the crosslinkable polymerizable monomer include

a photopolymerization initiator, such as a (bis)acylphosphine oxide compound, a thioxanthone compound or a quaternary ammonium salt of a thioxanthone compound, a ketal compound, an α-diketone compound, a coumarin compound, an anthraquinone compound, a benzoyl alkyl ether compound, and an α-aminoketone-based compound;
a polymerization accelerator, such as an aldehyde compound, a thiol compound, and an aminobenzoate ester compound;
an antibacterial substance, such as cetylpyridinium chloride, benzalkonium chloride, (meth)acryloyloxyldodecylpyridinium bromide, (meth)acryloyloxyhexadecylpyridinium chloride, (meth)acryloyloxydecylammonium chloride, triclosan, and 12-methacryloyloxydodecylpyridinium bromide; and
a monofunctional polymerizable monomer, such as 4-hydroxybutyl (meth)acrylate, 6-hydroxyhexyl (meth)acrylate, 8-hydroxyoctyl (meth)acrylate, 10-hydroxydecyl (meth)acrylate, and octafluoropentyl (meth)acrylate.

Specific examples of the (bis)acylphosphine oxide compound used as the photopolymerization initiator include 2,4,6-trimethylbenzoyl diphenylphosphine oxide, 2,4,6-trimethylbenzoyl methoxyphenylphosphine oxide, bis(2,4,6-trimethylbenzoyl)phenylphosphine oxide, and sodium 2,4,6-trimethylbenzoyl phenylphosphine oxide.

Specific examples of the α-diketone compound used as the photopolymerization initiator include diacetyl, benzil, camphorquinone, 2,3-pentanedione, 2,3-octanedione, 9,10-phenanthrenequinone, 4,4'-oxybenzyl, and acenaphthenequinone.

Specific examples of the aldehyde compound used as the polymerization accelerator include terephthalaldehyde and a benzaldehyde derivative. Examples of the benzaldehyde derivative include dimethylaminobenzaldehyde, p-methoxybenzaldehyde, p-ethoxybenzaldehyde, and p-n-octyloxybenzaldehyde.

Specific examples of the thiol compound used as the polymerization accelerator include 2-mercaptobenzoxazole, decanethiol, 3-mercaptopropyltrimethoxysilane, and thiobenzoic acid.

Specific examples of the aminobenzoate ester compound used as the polymerization accelerator include ethyl 4-(N,N-dimethylamino)benzoate, methyl 4-(N,N-dimethylamino)benzoate, n-butoxyethyl 4-N,N-(dimethylamino)benzoate, 2-[(meth)acryloyloxy]ethyl 4-(N,N-dimethylamino)benzoate, 4-(N,N-dimethylamino)benzophenone, and n-butyl

4-(N,N-dimethylamino)benzoate.

<Additional Component>

The dental composition may further contain one kind or two or more kinds of additional components. Examples of the additional component include a pH adjuster, a polymerization inhibitor, an ultraviolet ray absorbent, a thickener, a colorant (such as a dye and a pigment), a fluorescent agent, and a perfume. The dental composition may contain, for example, as the additional component, a fluoride ion releasing material, such as sodium fluoride, potassium fluoride, sodium monofluorophosphate, lithium fluoride, strontium fluoride, and ytterbium fluoride. The dental composition may contain, for example, a metal compound, such as silver oxide and aluminum hydroxide.

In the case where the dental composition contains the additional component, the content of the additional component is not particularly limited, as long as exerting the effects of the present invention, and may be such an amount that can provide the necessary effect, and the content thereof is preferably 0.01 to 1% by mass, more preferably 0.05 to 0.5% by mass, and further preferably 0.07 to 0.5% by mass, based on 100% by mass of the dental composition, from the standpoint of facilitating the sufficient securement of the effectiveness of the strengthening of the tooth substance and the treatment of initial caries.

In the case where the additional component is an organic compound, the content thereof is preferably 5% by mass or less, more preferably 3% by mass or less, further preferably 1% by mass or less, still further preferably 0.1% by mass or less, and still more further preferably 0.05% by mass or less, based on 100% by mass of the dental composition. In other words, in one embodiment of the present invention, in the case where the additional component is an organic compound, the content thereof is preferably 0 to 5% by mass, more preferably 0 to 3% by mass, further preferably 0 to 1% by mass, still further preferably 0 to 0.1% by mass, and still more further preferably 0 to 0.05% by mass, based on 100% by mass of the dental composition.

The dental composition according to one embodiment of the present invention may contain a zinc ion as the additional component, as long as exerting the effects of the present invention, but preferably substantially does not contain a zinc ion from the standpoint of further facilitating the effects of the present invention.

In the case where the dental composition substantially does not contain a zinc ion, it is estimated that in the formation process of the deposit caused by the dental composition, the influence of a zinc ion on at least one of the function of precipitating the compound (a) having an acidic group, the calcium ion (b1), and the inorganic component of the demineralized tooth substance through compounding, and the function of depositing on the tooth substance surface can be reduced, and thereby the deposit can be more easily formed thereon.

The zinc ion is derived from a zinc compound. The zinc compound is dissolved with the compound (a) having an acidic group, so as to allow the dental composition to contain the zinc ion.

The zinc ion is preferably derived from at least one zinc compound selected from the group consisting of ZnO and a compound represented by the following formula (VI):

$$(Zn^{2+})_e \cdot (Q^{r-})_f \qquad (VI)$$

wherein in the formula (VI), $Q^{r-}$ represents an anion as a counter ion of the zinc ion, r represents an integer of 1, 2, or 3, and e and f each represent an integer of 1, 2, or 3, in which e, f, and r have a relationship of 2e = rf.

More specific examples of the zinc compound include at least one zinc compound selected from ZnO, $ZnCl_2$, $Zn(OH)_2$, and $ZnF_2$.

The expression "substantially does not contain" a zinc ion herein means that the content of the zinc ion is 5% by mass or less, preferably 3% by mass or less, more preferably 1% by mass or less, further preferably 0.1% by mass or less, and still further preferably 0.05% by mass or less, based on 100% by mass of the dental composition. In other words, the expression "substantially does not contain a zinc ion" in the description herein means that the content of the zinc ion is 0 to 5% by mass, preferably 0 to 3% by mass, more preferably 0 to 1% by mass, further preferably 0 to 0.1% by mass, and still further preferably 0 to 0.05% by mass, based on 100% by mass of the dental composition.

In the description herein, the content of the zinc ion in the dental composition can be calculated based on the blending amount of the zinc compound generating the zinc ion, or can also be obtained through measurement with an ICP emission spectrometer.

[Production Method of Dental Composition]

The production method of the dental composition according to one embodiment of the present invention may include mixing the compound (a) having an acidic group, the calcium compound (b), the water (c), and the hydrophilic solvent (d) in any order, and the dental composition can be obtained by providing finally the state where the calcium compound (b) as a solid component is dissolved in the liquid mixture composition containing the compound (a) having an

acidic group, the water (c), and the hydrophilic solvent (d).

[Application of Dental Composition]

The dental composition can be delivered, for example, as a one-component type liquid, and can be applied to a tooth substance with a swab, a sponge chip, a small brush, an applicator brush, or the like. After applying, the transpirable component may be dried through air blowing with a three-way syringe or the like depending on necessity, so as to complete the treatment. Due to the convenient operative procedure, the stress experienced by both the practitioner and the patient is minimal even in treating multiple teeth.

The tooth substance strengthening function provided by the dental composition according to one embodiment of the present invention is particularly significant for dentin, and therefore the dental composition can be favorably used as a "dentin strengthening agent".

The dental composition according to one embodiment of the present invention can be favorably applied to the purposes of the prevention of occurrence of caries, the suppression of progression of initial caries, the suppression of secondary caries, and the suppression of hyperesthesia of dentin.

The present invention encompasses embodiments including various combinations of the aforementioned configurations within the scope of the technical concept of the present invention, as long as exerting the effects of the present invention.

Examples

The dental composition according to the present invention will be specifically described with reference to examples below, but the dental composition according to the present invention is not limited to the examples.

The abbreviations of the components used in Examples and Comparative Examples are as follows.

[Compound (a) having Acidic Group]

MHP: 6-methacryloyloxyhexyl dihydrogen phosphate
MDP: 10-methacryloyloxydecyl dihydrogen phosphate
M12P: 12-methacryloyloxydodecyl dihydrogen phosphate
4-MET: 4-methacryloyloxyethyl trimellitate

[Hydrophilic Solvent (d)]

HEMA: 2-hydroxyethyl methacrylate
DEAA: N,N-diethylacrylamide (compound represented by the formula (V))

[Additional Organic Compounds]

CPC: cetylpyridinium chloride
Bis-GMA: 2,2-bis[4-(3-methacryloyloxy-2-hydroxypropoxy)phenyl]propane
CQ: DL-camphorquinone
EDMB: ethyl 4-(N,N-dimethylamino)benzoate

[Preparation and Evaluation of Dental Compositions]

<Examples 1 to 13 and Comparative Examples 1 to 4>

The materials were mixed according to the formulations shown in Tables 1 and 2 to produce dental compositions of Examples and Comparative Examples. A specimen was produced with each of the compositions in the following manner, and the specimen was evaluated according to the following procedure.

The contents of the calcium ion (b1) shown in Tables 1 and 2 are derived from the calcium compound (b) shown in

Tables 1 and 2, and each are a value that is calculated from the blending amount of the calcium compound (b) blended in the examples.

[Evaluation of Tooth Substance Sealability, Acid Resistance, and Base Resistance]

<Preparation of Test Specimen>

The center of the buccal side of a healthy bovine tooth from which the dental root had been cut off and the dental pulp and the gums had been removed was trimmed by polishing with #80 polishing paper (silicon carbide polishing paper, available from Nihon Kenshi Co., Ltd.) using a rotary polisher ("Ecomet 4", available from Buehler, Ltd.) for exposing a flat surface of the dentin on the buccal side, thereby producing a dentin plate having a length of approximately 1.5 cm, a width of approximately 1 cm, and a thickness of approximately 2 to 3 mm.

The exposed dentin surface of the dentin plate was further polished with #1000 polishing paper (silicon carbide polishing paper, available from Nihon Kenshi Co., Ltd.) until the polishing marks formed with the #80 polishing paper disappeared.

Both ends in the length direction of the polished dentin plate were cut with a diamond cutter, thereby producing a test specimen having a length of approximately 1 cm, a width of approximately 0.8 cm, and a thickness of approximately 2 mm.

<Treatment with Dental Composition and Acid-Base Treatment>

On the exposed dentin surface of the test specimen prepared from the bovine tooth, the dental compositions prepared in Examples and Comparative Examples each were applied over the entire test surface by using a dental micro blush, and after air blowing and then immersing in water at 25°C, subjected to an ultrasonic treatment for 5 minutes with an sonicator ("Bransonic (registered trade name) 2510J-PTH", available from Branson Ultrasonics Corporation).

The test specimen having been treated with the dental composition was immersed in a 0.5 M acetic acid buffer solution having pH 4.5 at 25°C for 90 minutes, then taken out therefrom, and rinsed with running water for 30 seconds. After rinsing with water, the specimen was further immersed in a 5% sodium hypochlorite solution at 25°C for 20 minutes, then taken out therefrom, and rinsed with running water for 30 seconds, followed by drying by air blowing.

<SEM Observation>

The center of the surface of the specimen after subjecting to the acid-base treatment was observed with a scanning electron microscope (SEM) ("SU3500 type Scanning Electron Microscope", available from Hitachi High-technologies Corporation), and evaluated according to the following grading standard using 5-stage rating with "5" for the best state and "1" for the worst state. The SEM observation was performed under condition of an acceleration voltage of 15 kV and a magnification of 1,000.

The evaluation results are shown in Tables 1 and 2 below.

(Evaluation Standard)

5: The entire dentin surface was coated with the deposit.

4: The entire dentin surface was coated with the deposit, but the deposit was thin, and the presence of the dentinal tubules was confirmed at some locations.

3: An area having the deposit on the dentin surface and an area having dentinal tubules clearly observed existed as mixture.

2: Substantially no deposit on the dentin surface was observed, but the deposition inside the dentinal tubules was observed.

1: No deposit on the dentin surface was observed, and the dentinal tubules were opened.

[Table 1]

Table 1

| | Name of component | Unit | Example 1 | Example 2 | Example 3 | Example 4 | Example 5 |
|---|---|---|---|---|---|---|---|
| Compound (a) having acidic group | Dodecyl dihydrogen phosphate | part by mass | 30 | - | - | - | - |
| | Hexadecyl dihydrogen phosphate | part by mass | - | 30 | - | - | - |
| | MHP | part by mass | - | - | 30 | - | - |
| | MDP | part by mass | - | - | - | 30 | - |
| | M12P | part by mass | - | - | - | - | 30 |
| | 4-MET | part by mass | - | - | - | - | - |
| Calcium compound (b) | $Ca(OH)_2$ | part by mass | 1.6 | 1.3 | 1.6 | 1.3 | 1.3 |
| | $CaCl_2$ | part by mass | - | - | - | - | - |
| | CaO | part by mass | - | - | - | - | - |
| Calcium ion (b1) derived from calcium compound (b) (*1) | | part by mass | 0.87 | 0.70 | 0.87 | 0.70 | 0.70 |
| Water (c) | Water | part by mass | 35 | 35 | 35 | 35 | 35 |
| Hydrophilic solvent (d) | Ethanol | part by mass | 35 | 35 | 35 | - | 35 |
| | Isopropanol | part by mass | - | - | - | 35 | - |
| | HEMA | part by mass | - | - | - | - | - |
| | DEAA | part by mass | - | - | - | - | - |
| Additional components (except for organic compounds below) | NaF | part by mass | - | - | - | - | - |
| | $Zn(OH)_2$ | part by mass | - | - | - | - | - |
| | $ZnCl_2$ | part by mass | - | - | - | - | - |
| | $Ag_2O$ | part by mass | - | - | - | - | - |

(continued)

| | Name of component | Unit | Example 1 | Example 2 | Example 3 | Example 4 | Example 5 |
|---|---|---|---|---|---|---|---|
| Additional organic compounds | CPC | part by mass | - | - | - | - | - |
| | Bis-GMA | part by mass | - | - | - | - | - |
| | CQ | part by mass | - | - | - | - | - |
| | EDMB | part by mass | - | - | - | - | - |
| base gram equivalent/acid gram equivalent | | | 0.20 | 0.19 | 0.21 | 0.19 | 0.20 |
| SEM observation result of dentin surface after acid-base treatment | | | 5 | 5 | 5 | 5 | 5 |
| (*1): Calculated value from blending amount of calcium compound (b)<br>(*2): "5" for the best state, "1" for the worst state, "4", "3", and "2" for the intermediate states in stepwise manner | | | | | | | |

Table 1 (continued)

| | Name of component | Unit | Example 6 | Example 7 | Example 8 | Example 9 | Example 10 |
|---|---|---|---|---|---|---|---|
| Compound (a) having acidic group | Dodecyl dihydrogen phosphate | part by mass | - | - | - | - | 25 |
| | Hexadecyl dihydrogen phosphate | part by mass | - | 30 | 30 | - | - |
| | MHP | part by mass | - | - | - | - | - |
| | MDP | part by mass | 30 | - | - | - | - |
| | M12P | part by mass | - | - | - | - | - |
| | 4-MET | part by mass | - | - | - | 30 | - |
| Calcium compound (b) | $Ca(OH)_2$ | part by mass | 1.3 | - | - | 1.3 | 1.3 |
| | $CaCl_2$ | part by mass | - | 3.1 | - | - | - |
| | CaO | part by mass | - | - | 1.1 | - | - |
| Calcium ion (b1) derived from calcium compound (b) (*1) | | part by mass | 0.70 | 1.1 | 0.79 | 0.70 | 0.70 |
| Water (c) | Water | part by mass | 35 | 35 | 35 | 35 | 35 |

(continued)

| | Name of component | Unit | Example 6 | Example 7 | Example 8 | Example 9 | Example 10 |
|---|---|---|---|---|---|---|---|
| Hydrophilic solvent (d) | Ethanol | part by mass | - | - | 35 | 35 | 40 |
| | Isopropanol | part by mass | - | - | - | - | - |
| | HEMA | part by mass | 35 | - | - | - | - |
| | DEAA | part by mass | - | 35 | - | - | - |
| Additional components (except for organic compounds below) | NaF | part by mass | - | 0.1 | - | - | - |
| | $Zn(OH)_2$ | part by mass | - | - | 0.1 | - | 0.2 |
| | $ZnCl_2$ | part by mass | - | - | - | 0.1 | - |
| | $Ag_2O$ | part by mass | - | - | - | - | - |
| Additional organic compounds | CPC | part by mass | - | - | - | - | - |
| | Bis-GMA | part by mass | - | - | - | - | - |
| | CQ | part by mass | - | - | - | - | - |
| | EDMB | part by mass | - | - | - | - | - |
| base gram equivalent/acid gram equivalent | | | 0.19 | 0.30 | 0.21 | 0.19 | 0.20 |
| SEM observation result of dentin surface after acid-base treatment | | | 5 | 5 | 4 | 4 | 3 |
| (*1): Calculated value from blending amount of calcium compound (b)<br>(*2): "5" for the best state, "1" for the worst state, "4", "3", and "2" for the intermediate states in stepwise manner | | | | | | | |

Table 1 (continued)

| | Name of component | Unit | Example 11 | Example 12 | Example 13 |
|---|---|---|---|---|---|
| Compound (a) having acidic group | Dodecyl dihydrogen phosphate | part by mass | - | - | - |
| | Hexadecyl dihydrogen phosphate | part by mass | 30 | - | - |
| | MHP | part by mass | - | - | - |
| | MDP | part by mass | - | - | - |
| | M12P | part by mass | - | 30 | 15 |
| | 4-MET | part by mass | - | - | - |
| Calcium compound (b) | $Ca(OH)_2$ | part by mass | 1.3 | 1.6 | 1.6 |
| | $CaCl_2$ | part by mass | - | - | - |
| | CaO | part by mass | - | - | - |

(continued)

| | Name of component | Unit | Example 11 | Example 12 | Example 13 |
|---|---|---|---|---|---|
| Calcium ion (b1) derived from calcium compound (b) (*1) | | part by mass | 0.70 | 0.87 | 0.87 |
| Water (c) | Water | part by mass | 35 | 35 | 40 |
| Hydrophilic solvent (d) | Ethanol | part by mass | 35 | 35 | 45 |
| | Isopropanol | part by mass | - | - | - |
| | HEMA | part by mass | - | - | - |
| | DEAA | part by mass | - | - | - |
| Additional components (except for organic compounds below) | NaF | part by mass | - | - | - |
| | $Zn(OH)_2$ | part by mass | - | - | - |
| | $ZnCl_2$ | part by mass | 0.3 | - | - |
| | $Ag_2O$ | part by mass | - | - | - |
| Additional organic compounds | CPC | part by mass | - | 5.4 | - |
| | Bis-GMA | part by mass | - | - | - |
| | CQ | part by mass | - | - | - |
| | EDMB | part by mass | - | - | - |
| base gram equivalent/acid gram equivalent | | | 0.19 | 0.25 | 0.50 |
| SEM observation result of dentin surface after acid-base treatment | | | 3 | 5 | 2 |
| (*1): Calculated value from blending amount of calcium compound (b)<br>(*2): "5" for the best state, "1" for the worst state, "4", "3", and "2" for the intermediate states in stepwise manner | | | | | |

[Table 2]

Table 2

| | Name of component | Unit | Comparative Example 1 | Comparative Example 2 | Comparative Example 3 | Comparative Example 4 |
|---|---|---|---|---|---|---|
| Compound (a) having acidic group | Dodecyl dihydrogen phosphate | part by mass | - | - | - | - |
| | Hexadecyl dihydrogen phosphate | part by mass | - | - | 26.6 | - |
| | MHP | part by mass | - | - | - | 31.6 |
| | MDP | part by mass | 30 | 15.2 | - | - |
| | M12P | part by mass | - | - | - | - |
| | 4-MET | part by mass | - | - | - | - |

(continued)

| | Name of component | Unit | Comparative Example 1 | Comparative Example 2 | Comparative Example 3 | Comparative Example 4 |
|---|---|---|---|---|---|---|
| Calcium compound (b) | $Ca(OH)_2$ | part by mass | 1.3 | 2.0 | 1.3 | - |
| | $CaCl_2$ | part by mass | - | - | - | - |
| | CaO | part by mass | - | - | - | - |
| Calcium ion (b1) derived from calcium compound (b) (*1) | | part by mass | 0.70 | 1.08 | 0.70 | - |
| Water (c) | Water | part by mass | 70 | 21.2 | 31 | 31.6 |
| Hydrophilic solvent (d) | Ethanol | part by mass | - | 21.2 | 31 | - |
| | Isopropanol | part by mass | - | - | - | 36.8 |
| | HEMA | part by mass | - | 42.4 | - | - |
| | DEAA | part by mass | - | - | - | - |
| Additional components (except for organic compounds below) | NaF | part by mass | - | - | - | - |
| | $Zn(OH)_2$ | part by mass | - | - | - | - |
| | $ZnCl_2$ | part by mass | - | - | - | - |
| | $Ag_2O$ | part by mass | - | - | - | 4.4 |
| Additional organic compounds | CPC | part by mass | - | - | - | - |
| | Bis-GMA | part by mass | - | 45.5 | 10 | - |
| | CQ | part by mass | - | 3.0 | - | - |
| | EDMB | part by mass | - | 1.5 | - | - |
| base gram equivalent/acid gram equivalent | | | 0.19 | 0.57 | 0.21 | - |
| SEM observation result of dentin surface after acid-base treatment | | | N.D. (*3) | N.D. (*4) | N.D. (*4) | 1 |

(*1): Calculated value from blending amount of calcium compound (b)
(*2): "5" for the best state, "1" for the worst state, "4", "3", and "2" for the intermediate states in stepwise manner
(*3): Unable to test due to phase separation of composition
(*4): Unable to test due to adhesion of composition to observation surface

It is understood from the results in Table 1 that the dental compositions of Examples 1 to 13, which each are the dental composition that contains the components (a), (b1), (c), and (d) and substantially does not contain a crosslinkable polymerizable monomer, are excellent in sealability of the tooth substance, particularly the dentin, and are capable of imparting favorable acid resistance and base resistance to the tooth substance surface. Accordingly, it is understood

therefrom that the dental compositions can be favorably used as a "dentin strengthening agent".

On the other hand, as understood from the results in Table 2, the composition prepared in Comparative Example 1 causes phase separation due to the absence of the hydrophilic solvent (d) contained, and cannot be subjected to the evaluation.

The compositions prepared in Comparative Examples 2 and 3 contain the crosslinkable polymerizable monomer, which is an organic compound other than the compound (a) having an acidic group and the hydrophilic solvent (d), and in coating the composition on the test specimen, is adhered to the dentin surface, which is the SEM observation surface, disabling the acid-base treatment and also disabling the SEM observation.

It is understood that the composition used in Comparative Example 4 does not contain the calcium ion (b1), and thus cannot impart favorable acid resistance and base resistance to the tooth substance surface.

Industrial Applicability

The dental composition of the present invention can achieve the strengthening of a tooth substance and the restoration of an initial demineralized tooth substance through a convenient operation while securing high aesthetics, and therefore can be favorably applied particularly to the preventive care for root surface caries and the restoration treatment of incipient caries.

## Claims

1. A dental composition comprising

   a compound (a) having an acidic group represented by the following formula (I):

   $$R^1\text{-}X_k \qquad (I)$$

   wherein in the formula (I), $R^1$ represents an organic group having 40 or less carbon atoms that has or does not have at least one (meth)acryloyloxy group, X represents an acidic group, and k represents an integer of 1 or 2, in which in the case where k is 2, two acidic groups X may be the same as or different from each other,
   a calcium ion (b1), water (c), and a hydrophilic solvent (d),
   substantially not comprising a crosslinkable polymerizable monomer that is an organic compound other than the compound (a) having an acidic group, and is an organic compound other than the hydrophilic solvent (d).

2. The dental composition according to claim 1, wherein the compound (a) having an acidic group is represented by the following formula (II):

   $$R^1\text{-}((A^{m-})_a\cdot(H^+)_b)_k \qquad (II)$$

   wherein in the formula (II), $R^1$ and k have the same meanings as defined in the formula (I), $A^{m-}$ represents an m-valent acid anion group derived from the acidic group X, m represents an integer of 1, 2, or 3, and a and b represent integers of 1, 2, or 3 that have a relationship of am = b.

3. The dental composition according to claim 1 or 2, wherein the compound (a) having an acidic group is represented by the following formula (III):

   $$R^2\text{-}R^3\text{-O-P(=O)(-OH)}_2 \qquad (III)$$

   wherein in the formula (III), $R^2$ represents a (meth)acryloyloxy group or hydrogen, and $R^3$ represents an alkylene group having 6 to 20 carbon atoms.

4. The dental composition according to any one of claims 1 to 3, wherein the calcium ion (b1) is derived from at least one calcium compound (b) selected from a group consisting of CaO and a compound represented by the following formula (IV):

   $$(Ca^{2+})_c\cdot(Y^{n-})_d \qquad (IV)$$

   wherein in the formula (IV), $Y^{n-}$ represents an anion as a counter ion of the calcium ion, n represents an integer of 1, 2,

or 3, and c and d each represent an integer of 1, 2, or 3, in which c, d, and n have a relationship satisfying an expression 2c = nd.

**5.** The dental composition according to any one of claims 1 to 4, wherein the calcium ion (b1) is derived from at least one calcium compound (b) selected from a group consisting of CaO, $CaCl_2$, $Ca(OH)_2$, and $CaF_2$.

**6.** The dental composition according to any one of claims 1 to 5, wherein the dental composition has a content of the component (c) of 1 to 90% by mass and a content of the component (d) of 1 to 90% by mass based on 100% by mass in total of the compound (a) having an acidic group, the water (c), and the hydrophilic solvent (d).

**7.** The dental composition according to any one of claims 1 to 6, wherein the dental composition has a content of the component (a) of 10 to 70% by mass, a content of the component (c) of 1 to 90% by mass, and a content of the component (d) of 1 to 90% by mass, based on 100% by mass in total of the compound (a) having an acidic group, the water (c), and the hydrophilic solvent (d).

**8.** The dental composition according to any one of claims 1 to 7, wherein the dental composition has a ratio of a base gram equivalent derived from the calcium compound (b) with respect to an acid gram equivalent derived from the compound (a) having an acidic group (base gram equivalent/acid gram equivalent) of less than 0.50.

**9.** The dental composition according to any one of claims 1 to 8, wherein the dental composition substantially does not comprise a zinc ion.

**10.** The dental composition according to any one of claims 1 to 9, wherein the dental composition is a dentin strengthening agent.

## INTERNATIONAL SEARCH REPORT

International application No.
**PCT/JP2024/003438**

**A. CLASSIFICATION OF SUBJECT MATTER**

***A61K 6/54***(2020.01)i; ***A61K 8/19***(2006.01)i; ***A61K 8/24***(2006.01)i; ***A61K 8/37***(2006.01)i; ***A61Q 11/00***(2006.01)i
FI: A61K6/54; A61K8/24; A61K8/37; A61K8/19; A61Q11/00

According to International Patent Classification (IPC) or to both national classification and IPC

**B. FIELDS SEARCHED**

Minimum documentation searched (classification system followed by classification symbols)

A61K6/54; A61K8/19; A61K8/24; A61K8/37; A61Q11/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2015-140299 A (TOKUYAMA DENTAL CORP.) 03 August 2015 (2015-08-03) claims, paragraphs [0007], [0010]-[0029], [0044], [0082], [0086], example 12 | 1-10 |
| X | JP 2013-193971 A (TOKUYAMA DENTAL CORP.) 30 September 2013 (2013-09-30) claims, paragraphs [0002]-[0006], comparative example 13 | 1-4, 6-10 |
| Y | JP 2009-114070 A (TOKUYAMA DENTAL CORP.) 28 May 2009 (2009-05-28) claims, paragraphs [0021]-[0040], [0101], examples | 1-10 |
| Y | JP 2013-067615 A (SANCASTLE WORLDWIDE CORP.) 18 April 2013 (2013-04-18) claims, paragraphs [0002]-[0004], [0012]-[0017], [0026]-[0028], [0046], [0051], [0055], [0074], examples | 1-10 |
| Y | JP 2000-302622 A (MARUTOMO KK) 31 October 2000 (2000-10-31) claims, paragraphs [0031], [0033], [0040], [0041], examples | 1-10 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 March 2024** | **09 April 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/003438**

**C. DOCUMENTS CONSIDERED TO BE RELEVANT**

| Category* | Citation of document, with indication, where appropriate, of the relevant passages | Relevant to claim No. |
|---|---|---|
| Y | JP 2001-508433 A (SOCIEDAD LIMITADA PARA EL DESARROLLO CIENTIFICO AP) 26 June 2001 (2001-06-26)<br>claims, examples | 1-10 |
| Y | JP 2012-085857 A (SUN MEDICAL CO., LTD.) 10 May 2012 (2012-05-10)<br>claims, paragraphs [0021]-[0024], [0041], examples | 1-10 |
| A | JP 2012-020975 A (TOKUYAMA DENTAL CORP.) 02 February 2012 (2012-02-02)<br>entire text, all drawings | 1-10 |
| A | JP 2014-189488 A (KURARAY NORITAKE DENTAL INC.) 06 October 2014 (2014-10-06)<br>entire text, all drawings | 1-10 |
| A | JP 2013-151545 A (THREE M INNOVATIVE PROPERTIES CO.) 08 August 2013 (2013-08-08)<br>entire text, all drawings | 1-10 |

Form PCT/ISA/210 (second sheet) (July 2022)

**INTERNATIONAL SEARCH REPORT**
**Information on patent family members**

International application No.
**PCT/JP2024/003438**

| Patent document cited in search report | Publication date (day/month/year) | Patent family member(s) | Publication date (day/month/year) |
|---|---|---|---|
| JP 2015-140299 A | 03 August 2015 | (Family: none) | |
| JP 2013-193971 A | 30 September 2013 | (Family: none) | |
| JP 2009-114070 A | 28 May 2009 | (Family: none) | |
| JP 2013-067615 A | 18 April 2013 | US 2013/0078292 A1<br>claims, paragraphs [0012]-[0017], [0026]-[0038], examples<br>WO 2013/040952 A1<br>EP 2578272 A1<br>CN 103006701 A | |
| JP 2000-302622 A | 31 October 2000 | (Family: none) | |
| JP 2001-508433 A | 26 June 2001 | US 6413498 B1<br>claims, examples<br>WO 1998/030191 A1<br>EP 968700 A1<br>CN 1244112 A | |
| JP 2012-085857 A | 10 May 2012 | (Family: none) | |
| JP 2012-020975 A | 02 February 2012 | (Family: none) | |
| JP 2014-189488 A | 06 October 2014 | (Family: none) | |
| JP 2013-151545 A | 08 August 2013 | US 2009/0208909 A1<br>entire text, all drawings<br>WO 2006/055327 A1<br>EP 1819313 A1<br>CA 2587275 A<br>KR 10-2012-0134150 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2006347943 A
- JP 2019099481 A